(19) **Europäisches Patentamt / European Patent Office / Office européen des brevets**

(11) **EP 1 539 821 B1**

(12) **FASCICULE DE BREVET EUROPEEN**

(45) Date de publication et mention de la délivrance du brevet:
**13.10.2010 Bulletin 2010/41**

(21) Numéro de dépôt: **03750846.2**

(22) Date de dépôt: **25.07.2003**

(51) Int Cl.:
*C07K 16/20* (2006.01)     *A61K 39/008* (2006.01)

(86) Numéro de dépôt international:
**PCT/FR2003/002358**

(87) Numéro de publication internationale:
**WO 2004/011500 (05.02.2004 Gazette 2004/06)**

(54) **ANTICORPS D'ISOTYPES IGG2 ANTI ANTIGENES D'EXCRETION SECRETION DE PROMASTIGOTES OU D'AMASTIGOTES DE LEISHMANIA**

IGG2 ANTIKÖRPER GEGEN EXKRETION SEKRETION ANTIGENEN VON LEISHMANIA PROMASTIGOTEN ODER AMASTIGOTEN

IGG2 ISOTYPE ANTIBODIES ANTI-ANTIGENS OF SECRETION-EXCRETION OF LEISHMANIA PROMASTIGOTE OR AMASTIGOTE

(84) Etats contractants désignés:
**AT BE BG CH CY CZ DE DK EE ES FI FR GB GR HU IE IT LI LU MC NL PT RO SE SI SK TR**

(30) Priorité: **26.07.2002 FR 0209506**

(43) Date de publication de la demande:
**15.06.2005 Bulletin 2005/24**

(73) Titulaire: **Bio Veto Tests, BVT (SARL)**
**83500 La Seyne sur Mer (FR)**

(72) Inventeurs:
- **PAPIEROK, Gérard**
  **83400 Hyères (FR)**
- **VICENS, Serge**
  **F-13180 Gignac la Nerthe (FR)**
- **LEMESRE, Jean-Loup,**
  **138, rue de Lodève**
  **34000 Montpellier (FR)**

(74) Mandataire: **Marek, Pierre**
**28, rue de la Loge**
**B.P. 42413**
**13215 Marseille Cedex 02 (FR)**

(56) Documents cités:
**EP-A- 0 301 961     EP-A- 1 425 301**

- **DEPLAZES P ET AL:** "Specific IgG1 and IgG2 antibody responses of dogs to Leishmania infantum and other parasites." PARASITE IMMUNOLOGY. ENGLAND SEP 1995, vol. 17, no. 9, septembre 1995 (1995-09), pages 451-458, XP008018879 ISSN: 0141-9838
- **JIMÉNEZ-RUIZ A ET AL:** "Cloning, sequencing, and expression of the PSA genes from Leishmania infantum." EUROPEAN JOURNAL OF BIOCHEMISTRY / FEBS. GERMANY 15 JAN 1998, vol. 251, no. 1-2, 15 janvier 1998 (1998-01-15), pages 389-397, XP001159173 ISSN: 0014-2956
- **KEMP M ET AL:** "The Leishmania promastigote surface antigen-2 (PSA-2) is specifically recognised by Th1 cells in humans with naturally acquired immunity to L. major." FEMS IMMUNOLOGY AND MEDICAL MICROBIOLOGY. NETHERLANDS MAR 1998, vol. 20, no. 3, mars 1998 (1998-03), pages 209-218, XP002245424 ISSN: 0928-8244
- **CIBRELUS P ET AL:** "Secreted antigens of the amastigote and promastigote forms of Leishmania infantum inducing a humoral response in humans and dogs." PARASITE (PARIS, FRANCE) FRANCE JUN 1999, vol. 6, no. 2, juin 1999 (1999-06), pages 121-129, XP008007414 ISSN: 1252-607X

**EP 1 539 821 B1**

• RIERA C ET AL: "Serological and parasitological follow-up in dogs experimentally infected with Leishmania infantum and treated with meglumine antimoniate." VETERINARY PARASITOLOGY. NETHERLANDS JUL 1999, vol. 84, no. 1-2, juillet 1999 (1999-07), pages 33-47, XP002245423 ISSN: 0304-4017

**Description**

La présente invention concerne la détection et l'utilisation d'anticorps d'isotype $IgG_2$ spécifiques d'antigènes d'excrétion sécrétion de *Leishmania* permettant :

- la mise en évidence d'une immunité à médiation cellulaire dépendant de lymphocytes T de type Th1,
- le suivi de la réponse immune après vaccination ou traitement,
- l'évaluation de l'efficacité d'un traitement chimiothérapeutique et/ou immunothérapeutique, chez les mammifères et en particulier chez l'homme, les canidés, les félidés et les équidés,
- l'évaluation d'un état de résistance à la leishmaniose,
- l'établissement d'une immunothérapie.

La présente invention concerne également :

- la mise en évidence d'une réponse humorale spécifique représentée par des IgG d'isotype $IgG_2$ chez le chien et/ou d'autres isotypes chez d'autres mammifères, réponse humorale reliée à une réponse à médiation cellulaire dépendant de lymphocytes T de type Th1,
- la détection de ces IgG spécifiques du parasite *Leishmania sp* en utilisant des antigènes d'Excrétion Secrétion (ES) parfaitement définis et
- le rôle neutralisant de ces IgG caractérisé par l'inhibition de la prolifération des formes amastigotes ou promastigotes de *Leishmania.*

Le complexe vaccinal thérapeutique constitué d'ES produits dans un milieu axénique et asérique défini tel que décrit dans la demande de Brevet d'Invention française N°01/07606 du 11 Juin 2001 induit une immunostimulation du système lymphocytaire de type Th1 assurant la mise en place d'une immunité protectrice. Cette immunité à médiation cellulaire qui se caractérise par une activation des lymphocytes et des macrophages et par la synthèse de cytokines spécifiques de la voie Th1, est reliée à une immunité humorale caractérisée par la production d'IgG spécifiques neutralisant d'isotypes particuliers.

On peut diviser de manière très schématique les réponses immunitaires en deux grandes catégories qualitativement distinctes, les réponses humorales qui mettent en jeu la production d'anticorps par les lymphocytes B, et les réponses cellulaires (réaction d'hypersensibilité retardée, réactions cytotoxiques) pour lesquelles les cellules effectrices sont des lymphocytes T. Il apparaît que dans la majorité des modèles expérimentaux et des situations cliniques étudiées, les cytokines d'origine lymphocytaire sont essentiellement produites par les cellules T auxiliaires CD4+ (ou helper) dont le rôle est de moduler ou de réguler l'immunité humorale et cellulaire et qui reconnaissent l'antigène en association avec les molécules de classe II du complexe majeur d'histocompatibilité. Un concept majeur a émergé en 1985 lorsque T.Mosmann et R. Coffman ont proposé que les cellules lymphocytaires T CD4+ exprimant des fonctions auxiliaires étaient en fait hétérogènes. Ainsi, grâce à l'étude de clones lymphocytaires T CD4+ de souris, cultivés à long terme, ces auteurs ont décrit l'existence de deux sous-populations majeures que l'on peut distinguer grâce à leur profil de sécrétion de cytokines à savoir les cellules Th1 (pour T helper de type 1) et les cellules Th2 (pour T helper de type 2).

Prenons comme exemple la leishmaniose, qui est une infection parasitaire endémique voire épidémique de régions tropicales et subtropicales du monde. Les *leishmania*, protozoaires flagellés de la famille des trypanosomatidae et du genre *Leishmania,* sont les agents pathogènes responsables de ces maladies.

Les nombreuses études portant sur les réponses immunitaires au cours des leishmanioses murines expérimentales ont conduit à la démonstration du rôle prépondérant de l'immunité à médiation cellulaire et de l'existence d'une dualité de la réponse immunologique. Il existe fondamentalement deux types de réponses à l'encontre des leishmanies : l'une qualifiée de « sensibilité », l'autre de « résistance ». C'est par le biais des lymphokines qu'elles sécrètent que les différentes sous-populations de lymphocytes T (CD4+) limitent ou exacerbent l'infection. Il a ainsi été démontré que la sous-population de lymphocytes T auxiliaires de type Th1 (producteur d'interféron gamma et d'interleukine 2) était capable d'éliminer les formes amastigotes intracellulaires par le biais de l'activation des macrophages (Reiner S.L et al., Annu Rev Immunol, 1995, 13, 151-177. Review). Inversement, la sous-population de lymphocytes T auxiliaires de type Th2 (producteur d'interleukine 4) est responsable de l'exacerbation de la maladie.

Chez l'homme, certains faits sont de nature comparable. Chez le chien (hôte naturel « réservoir » réceptif au cycle évolutif de *L.infantum*), la dualité de la réponse immunologique existe vraisemblablement. Seule une étude menée par Pinelli et al. (Infect. Immun., 62 :229, 1994) sur des animaux expérimentalement et naturellement infectés par *L.infantum,* a permis de montrer que l'asymptomatisme du chien (état clinique fréquemment rencontré) s'accompagnait de l'absence d'une réponse humorale et du développement d'une immunité à médiation cellulaire de type Th1 avec une réaction d'hypersensibité de type retardée positive et des taux élevés d'interleukine 2 et de TNF-$\alpha$ circulant dans les liquides biologiques.

La polarisation des réponses immunitaires vers un phénotype Th1 ou Th2 a été associée à de nombreuses situations pathologiques.

Pour les infections à *Leishmania, Trypanozoma, Candida* et autres organismes intracellulaires comme *Mycobacterium et Listeria* une réponse Th1 corrèle avec la résistance au pathogène.

La présente invention consiste notamment à détecter des anticorps dont la production accompagne la polarisation d'une immunité à médiation cellulaire vers une voie de type Th1.

Selon les spécialistes comme Pinelli (Pinelli.E et al. Infect. Immun, 1994, 62:229-235) les chiens leishmaniens correspondant à l'activation du système lymphocytaire de type Th2 présentent une réponse anticorps élevée. Cette production accrue en anticorps correspond à une hyperprotéinémie et induit l'apparition d'immunocomplexes entraînant une atteinte rénale (augmentation de la créatine et de l'urée sanguine). Il a d'autre part été démontré (RIERA.C et al, vet parasitology, 1999, 84, 1-2, p33-47) que ce taux d'IgG totales diminuait chez des chiens expérimentalement infectés après traitement au glucantime, ce traitement n'induisant cependant qu'une récession temporaire des symptômes, les rechutes restant possibles et non prévisibles.

Néanmoins, certains travaux préliminaires chez l'homme (KAWANO. P et al, Parasite Immunol, 1995, 17, 451-458) et chez le chien (NIETO C.G et al, Vet Immunol and Immunopathology, 1999, 67, 117-130) annoncent que les isotypes d'IgG seraient des marqueurs de la dichotomie immunitaire Th1/Th2. D'ailleurs, certains auteurs (DEPLAZES. P et al, Parasite Immunology, 1995, 17, p451-458 /RIERA. C et al, vet parasitology, 1999, 84, 1-2, p33-47) ont étudié plus précisément l'importance du rapport isotypes $IgG_1/IgG_2$ chez le chien leishmanien avant et après traitement. Ces $IgG_1/IgG_2$ étaient spécifiques d'un antigène somatique total de promastigote, le taux d'$IgG_2$ restait constant avant et après traitement, par contre une baisse importante des $IgG_1$ était noté après traitement.

Toutefois, la spécificité de ces isotypes à un antigène ou à un épitope parfaitement déterminé n'a jamais été établie ainsi que le rôle de ces isotypes.

Les chiens ayant reçu le complexe vaccinal thérapeutique décrit dans la demande de brevet d'invention française N° 01/07606 précitée et ainsi protégés, présentent des taux significatifs d'$IgG_2$ spécifiques des protéines excrétées sécrétées, ce qui est conforme à l'expansion préférentielle de lymphocytes T de type Th1. A contrario, les chiens ayant reçu un placebo ainsi que les chiens leihmaniens ne présentent pas ces $IgG_2$ spécifiques.

Les antigènes d'excrétion sécrétion du protozaire *Toxoplasma gondii* (brevet EP 0 301 961 A) ont été étudiés comme vaccins et produits thérapeutiques, mais ces antigènes n'ont jamais été corrélés à des IgG d'isotype particulier ayant un rôle bien défini. D'autre part des auteurs (CIBRELUS.P et al, Parasite, 1999, 6 n°2, p121-129) ont démontré que les chiens naturellement infectés présentaient des IgG totales envers les antigènes d'excrétion sécrétion de *Leishmania infantum* mais là aussi aucune corrélation n'a été établie avec des immuglobulines d'isotype particulier à rôle bien défini.

La présente invention a également pour but de démontrer que les immunoglobulines de classes $IgG_2$ et des sous-classes correspondantes, reliés au système Th1 sont spécifiques d'un antigène parfaitement défini : les antigènes d'excrétion secrétion de *Leishmania* (ES) et ont une activité neutralisante envers la prolifération des amastigotes et promastigotes de *Leishmania sp.*

Ces $IgG_2$ spécifiques induites chez les chiens immunisés avec des antigènes d'excrétion sécrétion n'existent pas chez les mammifères naturellement infectés. Elles sont donc différentes de celles citées dans les documents "Riera" et "Deplazes" susmentionnés qui décrivent des IgG de chiens naturellement leishmaniens.

Ces $IgG_2$ sont plus précisément spécifiques d'une protéine majeure excrétée sécrétée par les 2 stades de Leishmania sp de la famille des PSA « Protein Surface Antigen » correspondant à un intervalle de masse moléculaire de 52 à 58 Kda. Cet immunogène majeur présente un épitope « immunologiquement silencieux » lors d'une infection par des leishmanies chez l'homme ou le chien. Cet épitope situé dans la région carboxyterminale conservée est reconnu par les IgG de chien. Des auteurs (KEMP. M et al, FEMS Immunol and med microbiology, Netherlands, 1998, 20 n°3, p209-218) ont démontré l'existence d'une glycoprotéine de surface PSA-2 chez *Leishmania major* responsable de leishmaniose cutanée chez l'homme et la souris. Cette glycoprotéine qui correspond à un antigène de surface constitutif de la forme promastigote du parasite et non d'antigènes d'excrétion sécrétion, induit un état immunitaire de type Th1 chez la souris. D'autres équipes (JIMENEZ-RUIZ.A et al, Europ J of Biochemistry / FEBS Germany, 1998, 251 n°1-2, p389-397) démontre qu'une PSA de promastigote de *Leishmania infantum* est un immunogène majeur des cellules B chez les chiens naturellement infectés. Dans les 2 cas, la spécificité d'$IgG_2$ ayant une activité neutralisante n'a pas été mise en évidence. De plus, la réponse anticorps lors d'une infection naturelle selon JIMENEZ-RUJZ.A et al est essentiellement dirigée contre les motifs répétés riches en Leucine, et non contre la partie carboxyterminale, de l'antigène majeur de surface de *Leishmania infantum* (et donc non d'un antigène d'excrétion sécrétion).

L'antigène majeur des produits d'excrétion sécrétion a été caractérisé par l'élaboration d'anticorps monoclonaux (AMC), et appartient à la famille des PSA et correspond à l'intervalle de masse moléculaire de 52 à 58 Kda. Deux banques d'expression d'ADNc des formes promastigotes et des formes amastigotes de Leishmania ont été criblées immunologiquement à l'aide de l'AMC F5 afin d'identifier les immunogènes majeurs des AES. Ceci a permis d'isoler l'$ADN_C$ d'un clone donné qui code une protéine excrétée sécrétée de la famille des PSA.

Ces protéines PSA présentent des domaines répétés riches en leucine caractéristiques.

L'analyse des séquences $ADN_C$ révèle une séquence peptidique correspondant exactement à l'extrémité COOH-terminale des PSA isolées.

Au moyen d'une protéine recombinante codant pour la partie carboxyterminale des PSA, il a été démontré que les patients ou les chiens atteints de leishmaniose sont incapables de produire des anticorps contre le ou les épitopes portés par la protéine recombinante, alors qu'ils produisent des anticorps contre les antigènes ES natifs, donc contre d'autres épitopes présents sur les PSA natives. Par contre les chiens immunisés avec les AES de promastigotes de *Leishmania infantum,* et protégé contre la leishmaniose viscérale, présentent des anticorps d'isotype IgG2 spécifiques de la partie carboxyterminale.

La présente invention a également pour but de démontrer qu'après chimiothérapie ou immunothérapie, les IgG2 de chien spécifiques des antigènes ES de Leishmania et notamment de la partie carboxyterminale de la PSA apparaissent avec l'amélioration significative de l'état général de chiens atteints de Leishmaniose.

Comme toutes les maladies à transmission vectorielle, les leishmanioses se caractérisent par un cycle évolutif qui est relativement simple puisqu'il se partage entre deux hôtes, mammifères et insectes (phlébotomes) et comprend deux formes principales :

- une forme flagellée appelée promastigote, présente dans le tube digestif du vecteur phlébotome où il se multiplie avant d'acquérir sa forme infectante pour l'hôte mammifère, encore appelée métacyclique ;
- une forme non flagellée appelée amastigote, présente chez l'hôte mammifère dont le chien et l'homme.

Afin d'étudier la synthèse spécifique d'anticorps $IgG_2$ chez le chien, 2 types d'expériences sont effectués après immunisation par le complexe vaccinal thérapeutique constitué d'ES: rôle de l'effet « in vitro » d'immunsérum de chiens sur les cinétiques de multiplication des différents stades du parasite *Leishmania infantum ;* dosage des $IgG_2$ spécifiques anti antigènes d'excrétion sécrétion de *Leishmania.*

Pour nos expériences, nous avons utilisé le complexe vaccinal composé d'antigènes d'excrétion sécrétion de promastigotes démontré dans la demande de brevet française N° 01/07606 susmentionnée.

Les chiens sont immunisés selon le schéma suivant :

| | 4 semaines | | | 4 semaines | | |
|---|---|---|---|---|---|---|
| 1ère injection sous cutanée | $\rightarrow$ | 2nde injection sous cutanée | | $\rightarrow$ | | prélèvement sanguin pour étude des immunsérums et des anticorps. |

Un autre type d'expérience est effectué : dosage des IgG2 spécifiques anti antigènes ES de Leishmania chez des chiens leishmaniens traités par chimiothérapie. Pour cette expérience, nous avons utilisé, comme antigène, la protéine recombinante codant pour la partie carboxyterminale des PSA.

**Les méthodes utilisées sont les suivantes :**

a. Matériel parasitaire :

La culture de promastigotes de *leishmania infantum* MON.1 est récoltée en phase stationnaire de croissance. Les parasites sont lavés 3 fois dans 15 ml de PBS stérile (centrifugation à 4°C à 5000 tours pendant 10 min) puis repris dans 1 ml de PBS.

La différenciation promastigote/amastigote et donc la multiplication des amastigotes nécessitent $0,5.10^6$ parasites par ml de milieux de culture MAA20 pH 5,8 en boîte de culture incubée à 37°C et sous 5% de CO2 (conditions représentatives de l'environnement phagolysosomial) ; la prolifération des promastigotes nécessite $10^6$ parasites par ml de milieux de culture RPMI 20% pH 7,2 en boîte de culture incubée à 25°C (conditions représentatives du phlébotome).

Un test de viabilité au bleu Trypan® à 0,4% en PBS ainsi qu'un comptage en cellule de Thomas doit être fait afin de resuspendre les parasites en PBS stérile tels qu'ils soient à $10^6$/ml.

b. Préparation des sérums:

Un aliquot de chaque sérum est décomplémenté par passage au bain-marie pendant 45 min à 56°C puis centrifugé 12 min à 12000 tours pour éviter les agglutinines.

Dans un premier temps, le taux sérique en anticorps n'étant pas connu, une gamme de dilution aléatoire est réalisée. Pour $10^6$ parasites, il faut mettre en contact 20$\mu$l de sérum :

- 1$^{er}$ essai sérum pur : 5.10$^6$ parasites (répartis en RPMI et MAA) soit (5.10$^6$ X 20)/10$^6$ = 100 $\mu$l de sérum pur.
- 2$^{ème}$ essai dilution au ½: 50 $\mu$l de sérum pur + 50 $\mu$l de Tampon PBS stérile.
- 3$^{ème}$ essai dilution au ¼: 25 $\mu$l de sérum pur + 75 $\mu$l de PBS stérile.
- 4$^{ème}$ essai dilution au 1/8: 12,5 $\mu$l de sérum pur + 87,5 $\mu$l de PBS stérile

c. Contact Promastigotes -Anticorps:

Les contacts sont réalisés dans des tubes à eppendorff de 1,5 ml où 5.10$^6$ parasites sont traités par 100 $\mu$l de sérum pendant 30 min sur la glace (temps minimum nécessaire à un promastigote pour infecter un macrophage). L'excès d'anticorps est ensuite lavé deux fois par 1 ml de PBS et par une centrifugation à 5000 tours de 10 min et cela à 2 reprises.

d. Analyses :

Un aliquot de 10$\mu$l de chaque essai est alors pris juste après contact pour réaliser un test de viabilité au bleu Trypan® et une numération en cellule de Thomas, l'excédent servant à la mise en culture à partir de laquelle un comptage est réalisé quotidiennement.

• **Prolifération des promastigotes** (milieu RPMI 20%)

Un comptage en cellule de Thomas à partir de chaque essai est réalisé quotidiennement pour suivre la cinétique de multiplication.
A partir d'un aliquot de chaque essai de culture, un frottis parasitaire fixé au méthanol puis coloré au May-Grunvald-Giemsa est réalisé quotidiennement où le pourcentage de forme promastigotes, sphéromastigotes et amastigotes est déterminé par le comptage de 100 parasites.

• **Prolifération des amastigotes** (milieu MAA 20)

A partir d'un second aliquot de chaque essai de culture, une numération en cellule de Thomas est effectuée afin de suivre la cinétique de multiplication.
Les calculs d'inhibition de croissance sont réalisés sur le modèle suivant :

Le nombre de parasites traités avec le sérum sain (= Ns) → 100% de croissance
Le nombre de parasites traités avec le sérum immunisé (=Ni) → x% de croissance

$$\% \text{ inhibition} = 100 - (Ni \times 100)/Ns$$

e. Etudes des anticorps :

Les anticorps totaux IgG des chiens antiLeishmania sont détectés par immunofluorescence classique utilisant des lames coatées par des promastigotes (méthode de référence sérologique pour la leishmaniose canine).
La détection des IgG2 spécifiques des ESP s'effectue par ELISA selon la technique de microtitration de Kweider et al (J.immunol, 1987, 138 :299) en utilisant les ESP comme antigènes et un conjugué anti IgG2.

f. Analyses effectuées chez les chiens immunisés avec les ESP :

Une étude complète d'inhibition de la prolifération des amastigotes et des promastigotes est effectuée sur 2 chiens :

Chienne MINON : Braque de Weymar femelle de 4 ans, vaccinée avec ESP
Chienne MUMA : Epagneul Breton, femelle de 7 ans, contrôle placebo

Un test de viabilité des promastigotes est effectué sur 6 autres chiens en étude expérimentale et 4 chiens en étude terrain (essai du complexe vaccinal ES en zone endémique) comparativement à 2 chiens témoins.

g. Etude des anticorps chez des chiens leishmaniens traités par chimiothérapie :

Pour la 3ème expérience, des sérums de 5 chiens atteints de leishmaniose viscérale et traités par du glucantime pendant 1 à 2 mois, provenant du Dr B ont été analysés.

h. Etudes des anticorps chez des chiens leishmaniens traités par immunothérapie avec les ESP :

Trois chiens leishmaniens ont été traités par injection sous-cutanée d'ESP (3 injections de 50 $\mu$g, chaque injection étant séparée de deux semaines).

Les anticorps totaux IgG des chiens anti Leishmania sont détectés par immunofluorescence et la détection des anticorps IgG2 spécifiques des ESP s'effectue par ELISA comme précédemment décrit (cf : e : étude des anticorps) mais en utilisant comme antigène la protéine recombinante codant pour la partie carboxyterminale des PSA.

**RESULTATS:**

*1. Etude sur la prolifération des promastigotes de L.infantum :*

• L'étude suivante est réalisée sur des parasites mis en contact avec un sérum de chien sain ou immunisé 4 fois avec des produits d'excrétion-sécrétion de promastigotes (ESP) et avant challenge.

Déterminée par un test au bleu Trypan®, la viabilité des promastigotes était de 100% avant contact avec le sérum. Après 30 min de contact, la viabilité avec le sérum sain était toujours de 100% à toutes les dilutions alors qu'avec le sérum immunisé aux ESP elle n'était plus que de 50% avec le sérum pur, 73% avec le sérum dilué au ½, 92% et 94% avec respectivement le sérum dilué au ¼ et 1/8.

Le tableau ci-après décrit l'évolution du pourcentage d'inhibition de la croissance des promastigotes après contact de 30 minutes avec sérum de chien immunisé (ESP) par rapport au sérum de chien sain.

| Pourcentage d'inhibition | 1er jour | 2ème jour | 3ème jour | 4ème jour | Moyenne |
|---|---|---|---|---|---|
| I pur/S pur | 93% | 92% | 98% | 73% | 89% |
| I ½/S½ | 94% | 94% | 84% | 90% | 90% |
| I ¼/S¼ | 79% | 60% | 45% | 0% | 46% |
| I 1/8/S 1/8 | ND | 81% | 90% | 81% | 84% |

**• Interprétation :**

L'inhibition de la croissance avec le sérum anti-ESP (chien MINON) par rapport au sérum sain approche les 90% quelle que soit la dose (mise à part pour la dilution au ¼, erreurs de manipulations ?). Il apparaît que la valeur seuil d'activité de ce sérum n'est pas atteint avec cette gamme de dilutions, du moins l'effet cytotoxique est puissant et la croissance des promastigotes est fortement ralentie à toutes les dilutions de sérums.

*2. Etude sur la prolifération des amastigotes de L. infantum*

• L'étude suivante est réalisée sur les cultures en MAA.20 pour différenciation en amastigotes, de promastigotes traités avec un sérum d'avant challenge de chien sain ou immunisé 4 fois avec des ESP.

Le tableau ci-après indique les pourcentages d'inhibition de la multiplication des amastigotes après 30 minutes de contact avec sérum de chien immunisé (ESP) par rapport au sérum de chien sain :

| % inhibition | 1er jour | 2éme jour | 3ème jour | Moyenne |
|---|---|---|---|---|
| Ip/Sp | 66% | 68% | 61% | 65% |
| I ½/S½ | 47% | 60% | 42% | 51% |
| I ¼/S¼ | 24% | 47% | 47% | 39% |
| I 1/8/S1/8 | 15% | 18% | 24% | 19% |

**• Interprétations :**

Une inhibition de la croissance est constatée sur la cinétique de croissance des amastigotes lorsque avant différenciation les promastigotes ont été mis en contact avec un sérum anti-ESP. La croissance des amastigotes se trouve alors ralentie et cela de façon proportionnelle à la concentration de sérum. Plus le sérum du chien immunisé aux ESP est concentré, plus la multiplication des amastigotes se trouve inhibée. Il y a donc un effet de dose dépendance.

*3. Résultats obtenus sur d'autres chiens : chiens immunisés par ESP et protégés contre infection expérimentale et infection naturelle.*

La moyenne de viabilité des promastigotes après contact avec du sérum de chien immunisé par le complexe vaccinal ESP est de 16,8% (5 chiens vaccinés) et de 72,9% (5 chiens plabebo) pour du sérum de chiens non immunisés. Aussi, des anticorps de chien immunisé non_reliés au complément sont cytotoxiques pour les promastigotes.

Le tableau ci-après indique les pourcentages de viabilité des promastigotes après contact, avec des sérums de chiens immunisés et non immunisés avec les ESP.

| Chiens analysés | T1* (Control) | T2* (Control) | Etudes expérimentales (infection expérimentale) | | | | | |
|---|---|---|---|---|---|---|---|---|
| | | | E | O | G | K | 7 | 16 |
| | | | Placebo | Placebo | Vaccin | Vaccin | Vaccin | Placebo |
| **Pourcentage de viabilité** | 100% | 100% | 85.7% | 61.9% | 22.4% | 10.1% | 12.8% | 76.2% |

| Chiens analysés | T1* (Control) | T2* (Control) | Etudes sur le terrain (infection naturelle) | | | |
|---|---|---|---|---|---|---|
| | | | HC 046 | HC 051 | LB 120 | RR 019 |
| | | | Placebo | Vaccin | Placebo | Vaccin |
| **Pourcentage de viabilité** | 100% | 100% | 66.7% | 15.7% | 73.8% | 22.9% |

• Chiens T1 et T2 : chiens sains non immunisés et n'ayant pas reçu de placebo.

*4. Corrélation entre la production d'IgG$_2$ anti ESP et l'effet des sérums sur la viabilité des promastigotes*

Selon le tableau ci-dessous (Corrélation IgG2 anti ESP/inhibition prolifération des promastigotes), la baisse du pourcentage de viabilité des promastigotes correspond à la présence d'IgG2 spécifiques du complexe vaccinal ESP.

| Chiens expérimentalement infectés | MINON | MUMA | E | O | G | K | 7 | 16 |
|---|---|---|---|---|---|---|---|---|
| IgG totales Par IF* | - | - | - | - | - | - | - | - |
| IgG2 spécifiques des ESP** par ELISA | **0.391** | 0.108 | 0.065 | 0.104 | **0.299** | **0.299** | **0.310** | 0.056 |
| Pourcentage de viabilité | 50% | 100% | 85.7% | 61.9% | 22.4% | 10.1% | 12.8% | 76.2% |

| Chiens naturellement infectés | HC 046 | HC 051 | LB 120 | RR 019 |
|---|---|---|---|---|
| IgG totales par IF* | - | - | - | - |
| IgG2 spécifiques de ESP** par ELISA | 0.054 | **0.321** | 0.065 | **0.386** |

(suite)

| Chiens naturellement infectés | HC 046 | HC 051 | LB 120 | RR 019 |
|---|---|---|---|---|
| Pourcentage de viabilité | 66.7% | 15.7% | 73.8% | 22.9% |
| * IF : Immunofluorescence : négative : taux ≤ 1/100 <br> ** $IgG_2$ : DO à 492 nm : les chiffres en gras correspondent à des résultats positifs | | | | |

*5. Etude des IgG2 chez des chiens leishmaniens traités par chimiothérapie :*

Les 5 chiens leishmaniens présentaient au début du traitement un taux élevé en IgG totale (≥ 1/200), ce taux restait fixe ou descendait à 1/50 4 mois après le début du traitement pour 4 chiens.

Le cinquième est décédé 2 mois après le traitement, il présentait une augmentation en IgG totales (1/200 au 1/800) et était négatif en $IgG_2$ dirigés contre la partie carboxyterminale des PSA.

Par contre, les 4 autres chiens guérissent et présentent des $IgG_2$ contre la partie carboxyterminale des PSA.

*6. Etude des $IgG_2$ chez des chiens leishmaniens traités par immunothérapie avec les ESP :*

Les trois chiens présentent en début de traitement un taux très élevé en IgG totales. Ce taux diminue 3 mois après le traitement. En parallèle de cette baisse d'IgG totales, les signes cliniques s'estompent et des IgG2 spécifiques du produit injecté apparaissent (voir tableau ci-dessous) :

| Chiens leishmaniens | | LAFAYETTE | JERRY | RODEO |
|---|---|---|---|---|
| **IgG totales par IF*** | Avant traitement | 1/800 | 1/3200 | 1/800 |
| | Après traitement | 1/200 | 1/400 | 1/100 |
| **IgG spécifique des ESP par ELISA**** | Avant traitement | 0,052 | 0,060 | 0,059 |
| | Après traitement | <u>0,361</u> | <u>0,327</u> | <u>0,359</u> |
| * IF : Immunofluorescence : négative taux ≤ 1/100 <br> ** $IgG_2$ : DO à 492 nm, les caractères soulignés correspondent à des résultats positifs. | | | | |

Il apparaît donc que la capacité à produire des anticorps $IgG_2$ contre cet épitope spécifique des PSA accompagne l'évolution vers la guérison des chiens. Ce sont donc bien les $IgG_2$ qui sont responsables de la lyse des amastigotes et promastigotes de *Leishmania sp* in vitro et de la neutralisation de leur prolifération.

L'analyse de sérums de 3 chiens leishmaniens traités par le complexe vaccinal ESP donne exactement le même type de résultat, c'est à dire une apparition d'$IgG_2$ spécifiques (de la partie carboxyterminale des PSA des ESP) corrélant avec une activité du sérum neutralisant la prolifération des promastigotes de *Leishmania infantum* « in vitro » et une immunité à médiation cellulaire de type Th1.

Ces $IgG_2$, chez le chien, ou autres isotypes particuliers chez les autres mammifères reliés à une immunité cellulaire de type Th1, sont détectables par diverses méthodes in vitro, par exemple : ELISA, DOT BLOT, WESTERN BLOT, IMMUNOCHROMATOGRAPHIE, IMMUNO NEPHELOMETRIE, RIA, IMMUNO PRECIPITATION, ELECTRO SYNERESE et tout autre méthode in vitro faisant intervenir un système de conjugué ou autres systèmes de visualisation de la réaction Ag-Ac. Ainsi, les immunoglobulines selon l'invention peuvent être utilisées pour un produit de diagnostic permettant de détecter un ou des épitopes portés par les extrémités $NH_2$ et COOH terminales de la Protein Surface Antigen excrétée sécrétée par *Leishmania sp.* Il peut par exemple être utilisé un système ELISA sur support plastic et un système WESTERN BLOT sur membranes de nitrocellulose ou autres polymères faisant intervenir un conjugué enzymatique. Des supports de latex peuvent également être utilisés. Des radio-isotopes, des molécules fluorescentes, des molécules luminescentes ou des particules de couleur peuvent être couplés à ces divers antigènes pour être utilisés comme conjugués. Des colloïdes métalliques peuvent également être utilisés. Par ailleurs, les antigènes d'excrétion sécrétion d'amastigotes ou de promastigotes de *Leishmania sp* et plus particulièrement la partie carboxyterminale de la Protein Surface Antigen sous forme purifiée ou protéine recombinante sont couplés à des grosses molécules de biotine, avidine, streptavidine ou tout autre protéine pour les rendre plus accessibles.

Ainsi, les anticorps d'isotype IgG2 et des sous classes correspondantes selon l'invention peuvent être utilisés chez les mammifères comme marqueurs d'un état immunitaire de type à médiation cellulaire dépendante de lymphocytes T et préférentiellement de lymphocytes T de type Th1, comme marqueurs de la résistance à la leishmaniose et aux infections à micro-organismes pathogènes intracellulaires chez les mammifères, comme marqueurs de la vaccination immunoprophylactique et immunothérapeutique pour les infections à micro-organismes pathogènes intracellulaires, et comme effecteurs d'immunothérapie pour les leishmanioses et les infections à micro-organismes pathogènes intracellulaires.

**Revendications**

1. Immunoglobulines de classes IgG$_2$, spécifiques des antigènes d'excrétion sécrétion de promastigotes ou d'amastigotes de *Leishmania sp* et capables de lyser les amastigotes et promastigotes de *Leishmania sp* in vitro et neutraliser leur prolifération, **caractérisées en ce qu'**elles sont spécifiques de la partie carboxyterminale de l'immunogène majeur excrété sécrété par des promastigotes ou des amastigotes de *Leishmania sp,* appartenant à la famille des *Protein Surface Antigen* et correspondant à un intervalle de masse moléculaire de 52 à 58 Kda

2. Immunoglobulines selon la revendication 1 **caractérisées en ce qu'**elles sont d'isotypes IgG$_2$ chez le chien, isotypes reliés à une immunité à médiation cellulaire dépendante des lymphocytes T de type Th1.

3. Utilisation des immunoglobulines selon la revendication 1 ou 2 comme marqueur d'une immunité à médiation cellulaire permettant notamment la mise en évidence d'une immunité à médiation cellulaire dépendante de lymphocytes T et préférentiellement de lymphocytes T de type Th1 chez les mammifères.

4. Utilisation des immunoglobulines selon la revendication 1 ou 2 comme marqueurs de la résistance à la leishmaniose et aux infections à micro-organismes pathogènes intracellulaires chez les mammifères.

5. Utilisation des immunoglobulines selon la revendication 1 ou 2 comme marqueurs de la vaccination immunoprophylactique et immunothérapeutique chez les mammifères pour les leishmanioses et les infections à micro-organismes pathogènes intracellulaires.

6. Immuglobulines selon la revendication 1 ou 2 pour l'utilisation comme effecteurs d'immunothérapie dans le cadre des leishmanioses et des infections à micro-organismes pathogènes intracellulaires chez les mammifères.

7. Utilisation des immunoglobulines selon la revendication 1 ou 2 pour un produit de diagnostic in vitro détectant un ou des épitopes portés par les extrémités NH$_2$

**Claims**

1. Immunoglobulins of classes IgG$_2$ specific to the excretion-secretion antigens of promastigotes or amastigotes of *Leishmania sp* and capable of lysing the amastigotes and promastigotes of *Leishmania sp* in vitro and neutralising their proliferation, **characterised in that** they are specific to the carboxyterminal part of the major immunogen excreted-secreted by promastigotes or amastigotes of *Leishmania sp,* belonging to the family of the *Protein Surface Antigens* and corresponding to a molecular mass range of 52 to 58 Kda.

2. Immunoglobulins according to claim 1 **characterised in that** they are isotypes IgG$_2$ in dogs, isotypes linked to cell-mediated immunity dependent on the T lymphocytes of Th1 type.

3. Use of the immunoglobulins according to claim 1 or claim 2 as a marker of cell-mediated immunity allowing in particular detection of a cell-mediated immunity dependent on T lymphocytes and preferably T lymphocytes of type Th1 in mammals.

4. Use of the immunoglobulins according to claim 1 or claim 2 as markers of resistance to leishmaniasis and infections with pathogenic intracellular microorganisms in mammals.

5. Use of the immunoglobulins according to claim 1 or claim 2 as markers of immunoprophylactic and immunotherapeutic vaccination in mammals for leishmaniases and pathogenic intracellular microorganism infections.

6. Immunoglobulins according to claim 1 or claim 2 for use as effectors of immunotherapy in the context of leishmaniases and pathogenic intracellular microorganism infections in mammals.

7. Use of the immunoglobulins according to claim 1 or claim 2 for an in vitro diagnostic product detecting one or more epitopes carried by the terminal ends $NH_2$ and COOH of the Protein Surface Antigen excreted-secreted by *Leishmania sp.*

**Patentansprüche**

1. Immunglobuline der Klassen $IgG_2$, spezifisch für Exkretions-/Sekretions-Antigene von Promastigoten oder Amastigoten von *Leishmania sp* und fähig, die Amastigoten und Promastigoten von *Leishmania sp* in vitro zu lösen und ihre Proliferation zu neutralisieren, **dadurch gekennzeichnet, dass** sie spezifisch für den CarboxyTerminus des Hauptimmunogens sind, das durch Exkretion/Sekretion von den Promastigoten oder Amastigoten der *Leishmania sp* abgeschieden wird, das zur Familie des *Protein Surface Antigen* gehört und einem Molekularmassenintervall von 52 bis 58 kDa entspricht.

2. Immunglobuline nach Anspruch 1, **dadurch gekennzeichnet, dass** sie dem $IgG_2$-Isotyp beim Hund entsprechen, wobei diese Isotypen mit einer zellvermittelten Immunität in Verbindung stehen, die von T-Lymphozyten des Typs Th1 abhängig ist.

3. Verwendung der Immunglobuline nach Anspruch 1 oder 2 als Marker einer zellvermittelten Immunität, die insbesondere den Nachweis einer zellvermittelten Immunität gestatten, die von T-Lymphozyten und bevorzugt von T-Lymphozyten des Typs Th1 bei Säugetieren abhängig ist.

4. Verwendung der Immunglobuline nach Anspruch 1 oder 2 als Marker für die Resistenz gegen Leishmaniose und Infektionen durch pathogene intrazelluläre Mikroorganismen bei Säugetieren.

5. Verwendung der Immunglobuline nach Anspruch 1 oder 2 als Marker für immunprophylaktische oder immuntherapeutische Impfung bei Säugetieren gegen Leishmaniosen und Infektionen durch pathogene intrazelluläre Mikroorganismen.

6. Immunglobuline nach Anspruch 1 oder 2 zur Verwendung als immuntherapeutische Wirkstoffe im Rahmen von Leishmaniosen und Infektionen durch pathogene intrazelluläre Mikroorganismen bei Säugetieren.

7. Immunglobuline nach Anspruch 1 oder 2 für ein Produkt zur in-vitro-Diagnose, das ein oder mehrere Epitope erfasst, die von den $NH_2$- und COOH-Termini des von *Leishmania sp* durch Exkretion/Sekretion abgeschiedenen Protein Surface Antigen getragen werden.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 0107606

- EP 0301961 A

**Littérature non-brevet citée dans la description**

- **Reiner S.L et al.** *Annu Rev Immunol,* 1995, vol. 13, 151-177
- **Pinelli et al.** *Infect. Immun.,* 1994, vol. 62, 229
- **Pinelli.E et al.** *Infect. Immun,* 1994, vol. 62, 229-235
- **RIERA.C et al.** *vet parasitology,* 1999, vol. 84 (1-2), 33-47
- **KAWANO. P et al.** *Parasite Immunol,* 1995, vol. 17, 451-458
- **NIETO C.G et al.** *Vet Immunol and Immunopathology,* 1999, vol. 67, 117-130

- **DEPLAZES. P et al.** *Parasite Immunology,* 1995, vol. 17, 451-458
- **RIERA. C et al.** *vet parasitology,* 1999, vol. 84 (1-2), 33-47
- **CIBRELUS.P et al.** *Parasite,* 1999, vol. 6 (2), 121-129
- **KEMP. M et al.** *FEMS Immunol and med microbiology,* 1998, vol. 20 (3), 209-218
- **JIMENEZ-RUIZ.A et al.** *Europ J of Biochemistry / FEBS Germany,* 1998, vol. 251 (1-2), 389-397
- **Kweider et al.** *J.immunol,* 1987, vol. 138, 299